(19) Europäisches Patentamt
European Patent Office
Office européen des brevets

(11) Veröffentlichungsnummer: **0 186 035 B1**

(12) # EUROPÄISCHE PATENTSCHRIFT

(45) Veröffentlichungstag der Patentschrift: **11.12.91**

(51) Int. Cl.$^5$: **C12N 9/10**, C12P 13/04, C12P 35/00, C12P 41/00, //(C12N9/10,C12R1:10)

(21) Anmeldenummer: **85115678.6**

(22) Anmeldetag: **10.12.85**

(54) **Neue D-Aminosäure-Transaminase und ihre Verwendung.**

(30) Priorität: **22.12.84 DE 3447023**

(43) Veröffentlichungstag der Anmeldung: **02.07.86 Patentblatt 86/27**

(45) Bekanntmachung des Hinweises auf die Patenterteilung: **11.12.91 Patentblatt 91/50**

(84) Benannte Vertragsstaaten: **AT BE CH DE FR GB IT LI LU NL SE**

(56) Entgegenhaltungen:
**EP-A- 0 135 846**
**DE-A- 2 219 454**

**BIOLOGICAL ABSTRACTS, Band 58, 1. November 1974, Seite 5529, Zusammenfassung Nr. 51393, Philadelphia, PA, US; PATEL et al.: "Homologous and hybrid complexes of anthranilate synthase from Bacillus species", & J. BACTERIOL 199(1): 220-227. Illus. 1974**

**JOURNAL OF BACTERIOLOGY, Band 69, 1955, Seiten 357-362, American Society of Microbiology; C.B. THORNE et al.: "Transamination of D-amino acids by Bacillus subtilis"**

(73) Patentinhaber: **HOECHST AKTIENGESELLSCHAFT**
**Postfach 80 03 20**
**W-6230 Frankfurt am Main 80(DE)**

(72) Erfinder: **Aretz, Werner, Dr.**
**Am Krummorgen 2**
**W-6233 Kelkheim (Taunus)(DE)**
Erfinder: **Sauber, Klaus, Dr.**
**Friedrich-Ebert-Strasse 15**
**W-6231 Schwalbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die Überführung von Cephalosporin C (CPC) in 7-Amino-cephalosporansäure (7-ACA) erfolgt technisch über die Stufe der α-Ketoadipinyl-Verbindung. In der deutschen Offenlegungsschrift 2 219 454 ist ein Verfahren beschrieben, bei dem dieser Schritt mit aktivierten Zellen von Trigonopsis variabilis unter aeroben Bedingungen bewirkt wird. Es handelt sich hier um eine oxidative Desaminierung, bei der als Nebenprodukt Wasserstoffperoxid gebildet wird. Das wirksame Enzym aus dieser Hefe, die D-Aminosäure-Oxidase, ist jedoch gegen Wasserstoffperoxid empfindlich, wodurch eine Mehrfachverwendung des Enzyms begrenzt wird.

Die Erfindung betrifft demgegenüber eine neue D-Aminosäure-Transaminase, die CPC mit α-Ketosäuren zu α-Ketoadipinyl-7-ACA und zur entsprechenden D-α-Aminosäure umsetzt. Es handelt sich hierbei also um eine Transaminierung, wobei die Aminogruppe von CPC nichtoxidativ in die Ketogruppe umgewandelt wird, weshalb auch kein Wasserstoffperoxid freigesetzt wird.

Das erfindungsgemäße Enzym wird von dem Bakterienstamm Bacillus licheniformis ATCC 9945 gebildet und kann aus den Zellen nach Aufschluß in an sich bekannter Weise isoliert werden, wobei die Umwandlung von CPC mit α-Ketosäuren zu α-Ketoadipinyl-7-ACA als Kriterum zur Auffindung der Fraktion dienen kann, in der sich das erfindungsgemäße Enzym befindet. Hierbei ist darauf zu achten, daß der genannte Stamm eine weitere D-Aminosäure-Transaminase bildet, die jedoch nicht befähigt ist, CPC umzusetzen.

Die Leistungsfähigkeit des erfindungsgemäßen Enzyms beschränkt sich jedoch nicht auf diese Umsetzung von CPC zu α-Ketoadipinyl-7-ACA, sondern es kann ganz allgemein D-α-Aminosäuren mit α-Ketosäuren transaminieren:

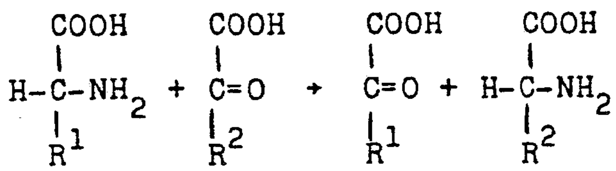

I II III IV

Als α-Ketosäuren (bzw. deren Salze) der allgemeinen Formel II setzt man zweckmäßig leicht zugängliche Verbindungen ein wie Brenztraubensäure, Oxalessigsäure, Phenylbrenztraubensäure, α-Ketoglutarsäure oder α-Ketoadipinsäure.

Wie sich schon aus der Umsetzung von CPC ergibt, ist bei den D-α-Aminosäuren der allgemeinen Formel I der Rest $R^1$ ziemlich unkritisch. Es können deshalb die üblichen Aminosäuren, aber beispielsweise auch Dipeptide eingesetzt werden.

Die Erfindung betrifft dementsprechend die Verwendung des neuen Enzyms zur Überführung von D-α-Aminosäuren in die entsprechenden α-Ketosäuren, aber auch zur Herstellung von D-Aminosäuren aus α-Ketosäuren. Erfindungsgemäß kann somit das neue Enzym auch zur Racematspaltung von D,L-Aminosäuren dienen, da hierbei die L-Aminosäure nicht angegriffen wird, die D-Aminosäure jedoch in die entsprechende α-Ketosäure überführt wird, die leicht abtrennbar ist. Zweckmäßig wählt man für diese Umsetzung eine α-Ketosäure, die zu einer D-α-Aminosäure führt, welche aus dem Reaktionsgemisch Leicht von der gewünschten L-Aminosäure abgetrennt werden kann.

In einer Ausgestaltung des letztgenannten Verwendungsverfahrens kann die L-Aminosäure aus dem Racemat mit Hilfe einer L-Aminosäure-Oxidase ebenfalls in die α-Ketosäure überführt werden. Hierfür kommen bevorzugt die Enzyme und das Verfahren aus der deutschen Patentanmeldung P 33 33 453.6 in Betracht.

Das erfindungsgemäße Enzym eignet sich weiterhin zum Nachweis von α-Ketosäuren neben L-Aminosäuren, insbesondere im Mikromaßstab.

Der Stamm Bacillus licheniformis ATCC 9945 ist allgemein zugänglich. Er wurde ursprünglich als Bacillus subtilis beschrieben (C.B. Thome, J. Bacteriology 69 (1955) 357 - 362). Ein bevorzugtes Verfahren zur Fermentierung dieses Stammes besteht darin, daß man ihn unter submersen Bedingungen in einem Nährmedium fermentiert, welches 0,1 bis 1 % Kohlenhydrate, 1 bis 5 % organische Stickstoffverbindungen und einen Induktor enthält, vorzugsweise eine Aminosäure in einer Konzentration von 0,1 bis 1 %, insbesondere D,L-Glutaminsäure. Die Fermentation erfolgt zweckmäßig bei 25 bis 35° C, insbesondere bei

etwa 30° C, bis zum Erreichen der stationären Phase, was nach etwa 24 Stunden der Fall ist.

Nach Abernten der Zellen werden diese aufgeschlossen, was mechanisch oder enzymatisch erfolgen kann. Der mechanische Aufschluß erfolgt in bekannter Weise, beispielsweise durch Ultraschall, French-Press oder ®DYNO-Mill (Willi Bachofen, Basel), jedoch vorzugsweise enzymatisch durch Behandlung mit Lysozym.

Der Überstand wird einer fraktionierten Ammoniumsulfatfällung mit anschließender Dialyse unterworfen und der Dialyserückstand über eine Ionenaustausch-Chromatographie weiter gereinigt. Hierbei werden Esterasen und β-Lactamasen abgetrennt und die D-Transaminasen in Fraktionen aufgespalten. Unter Verwendung einer hochauflösenden Diethylaminoethylmodifizierten Agarose erhält man das erfindungsgemäße Enzym als zweite Transaminase-aktive Fraktion.

In den folgenden Beispielen wird die Erfindung näher erläutert. Prozentangaben beziehen sich auf das Gewicht, wenn keine anderen Angaben gemacht sind.

Beispiel 1

Der Stamm Bacillus licheniformis ATCC 9945 wird auf Schrägröhrchen mit folgender Zusammensetzung gehalten:
0,3 % Bacto-Beef-Extract
0,5 % Bacto-Peptone
1,5 % Agar
(pH 7,0)
Nach einer Inkubationszeit von 2 - 3 Tagen bei 30° C werden die Sporen mit 10 ml physiologischer Kochsalzlösung abgeschwemmt und 1 ml dieser Suspension zum Animpfen von 100 ml Vorkultur folgender Zusammensetzung verwendet:
1 % Hefeextrakt
0,8 % Nutrient Broth
0,5 % Maltose
(pH 7,5)
Der Kolben wird bei 190 Upm auf einem Rotationsschüttler 24 Stunden bei 30° C inkubiert. Anschließend werden 50 ml dieser Vorkultur in 2 l fassende Erlenmeyerkolben mit je 500 ml Nährlösung der folgenden Zusammensetzung gebracht und als Hauptkultur für 24 Stunden bei 30° C und 190 Upm geschüttelt:
Hauptkulturmedium:
1 % Hefeextrakt
0,8 % Nutrient Broth
0,5 % D,L-Glutaminsäure
(pH 7,2)
Die D-Aminosäure-Transaminase-(DATA)-Aktivität erreicht in der stationären Wachstumsphase mit 0,5 U/g Zellen ihr Maximum.

Beispiel 2

Bacillus licheniformis ATCC 9945 wird wie in Beispiel 1 beschrieben in einer Vorkultur (500 ml) angezogen und nach 24 Stunden in einen 12 l-Fermenter mit 9 l des oben angegebenen Hauptkultur-Nährmediums verimpft. Die Fermentationsdauer beträgt bei 30° C, einer Belüftungsrate von 0,15 vvm und 300 Upm 22 - 26 Stunden. Die DATA-Aktivität entspricht der in Beispiel 1.

Beispiel 3

Der zur DATA-Isolierung notwendige Zellaufschluß erfolgt auf enzymatischem Wege. Dazu werden die in K-Phosphat-Puffer (pH 7,0, 10 mM + 10 μM Pyridoxalphosphat) aufgenommenen Zellen (0,5 g/ml) mit 1 mg Lysozym/ml Zellsuspension versetzt und 10 - 30 Minuten bei 190 UpM und 30° C inkubiert. Nach mikroskopischer Kontrolle wird der Inkubationsansatz zentrifugiert und der Überstand als Rohextrakt weiterverarbeitet.

Beispiel 4

Der nach Beispiel 3 erhaltene Rohextrakt wird einer fraktionierten Ammonsulfat-Fällung unterworfen: 208 ml des Rohextraktes werden ad 30 % der Sättigung mit Ammonsulfat versetzt und zentrifugiert. Der

Überstand wird ad 60 % der Sättigung weiter mit Ammonsulfat versetzt und abzentrifugiert. Der Niederschlag wird in 22 ml Phosphatpuffer (10 mM, pH 8,0), der 10 µM Pyridoxalphosphat enthält, aufgenommen und gegen den gleichen Puffer eine Nacht dialysiert.

Beispiel 5

15 ml eines nach Beispiel 4 hergestellten Enzymextraktes werden auf eine Säule (Totalvolumen 100 ml) mit einer Diethylaminoethyl-modifizierten Agarose (DEAE-Sepharose[R] FF von Pharmacia), die zuvor mit 10 mM Kaliumphosphatpuffer (pH 8,0 äquilibriert worden ist, aufgetragen. Mit einem Gradienten von 0,15 bis 0,5 M NaCl im Startpuffer wird bei pH 8,0 eluiert. Es eluieren 2 Aktivitäten (getestet mit D-Ala). Lediglilch die zweite Aktivität, die bei ungefähr 230 mM NaCl eluiert, ist CPC-aktiv. Diese Fraktion enthält weniger als 1 % Esterase-Aktivität im Vergleich zum Ausgangsmaterial.

Beispiel 6

Die nach Beispiel 5 erhaltenen D-Transaminase-aktiven Fraktionen werden zusammengefaßt. Durch Inkubation (bei 37° C) von CPC (20 mM) und α-Keto-glutarat (20 mM) in Phosphatpuffer (50 mM, pH 8,0) mit der Fraktion, die bei etwa 230 mM NaCl eluiert, wird α-Ketoadipinyl-7-ACA gebildet.

Eine Abtrennung kann durch HPLC auf "reversed phase"-Material (RP 18-Kieselgel, mit Octadecyl-Resten modifiziert, mobile Phase: 3 % Methanol) erfolgen (UV-Nachweis).

Die folgende Tabelle zeigt weitere Umsetzungen mit dem erfindungsgemäßen Enzym.

| Aminogruppen- | Aminogruppenakzeptoren | |
|---|---|---|
| Donatoren | α-Ketoglutarsäure | α-Ketoadipinsäure |
| D-Alanin | +++ | ++ |
| D-α-Aminoadipinsäure | +++ | |
| D-Asparaginsäure | +++ | +++ |
| D-Glutaminsäure | | ++ |
| D-Leucin | ++ | + |
| D-Lysin | + | - |
| D-Methionin | + | ++ |
| D-Phenylalanin | + | (+) |
| D-Prolin | (+) | (+) |

| | | |
|---|---|---|
| D-Serin | ++ | + |
| D-Tryptophan | + | + |
| D-Tyrosin | + | - |
| D-Valin | + | - |
| D-Cystin | + | (+) |
| D-Ala-D-Ala | + | - |

+++ sehr gute Umsetzung
++ gute Umsetzung
+ deutliche Umsetzung
(+) schwache Umsetzung
- keine Umsetzung

Als Aminogruppenakzeptoren können auch Pyruvat, Oxalacetat und Phenylpyruvat eingesetzt werden.

**Patentansprüche**

1. Verwendung von D-Aminosäure-Transaminase, aus Bacillus licheniformis ATCC 9945, zur Umsetzung von Cephalosporin C mit α-Ketosäuren zu α-Ketodipinyl-7-ACA und zur entsprechenden D-α-Aminosäure

2. Verfahren zur Herstellung von D-Aminosäure-Transaminase, dadurch gekennzeichnet, daß
   a) Bacillus licheniformis ATCC 9945 aerob fermentiert wird,
   b) die Biomasse vom Fermentationsmedium abgetrennt und aufgeschlossen wird und
   c) die Fraktion, die Cephalosporin mit α-Ketosäuren zu α-Ketoadipinyl-7-ACA umsetzt, isoliert wird.

3. Verwendung von D-Aminosäure-Transaminase, erhältlich durch Fermentation von Bacillus licheniformis ATCC 9945, Abtrennung und Aufschließen der Zellen und Isolierung der D-Aminosäure-Transaminase-Fraktion, zur Umsetzung von Cephalosporin C mit α-Ketosäuren zu α-Ketodipinyl-7-ACA.

**Claims**

1. The use of a D-amino-acid transaminase from Bacillus licheniformis ATCC 9945 for converting cephalosporin C with α-keto acids into α-ketoadipinyl-7-ACA and into the corresponding D-α-amino acid.

2. A process for the preparation of a D-amino-acid transaminase, which comprises
   a) Bacillus licheniformis ATCC 9945 being aerobically fermented,
   b) the biomass being removed from the fermentation medium and disrupted,
   c) the fraction which converts cephalosporin with α-keto acids into α-ketoadipinyl-7-ACA being isolated.

3. The use of the D-amino-acid transaminase obtainable by fermentation of Bacillus licheniformis ATCC 9945, removal and disruption of the cells and isolation of the D-amino-acid transaminase fraction for converting cephalosporin C with α-keto acids into α-ketoadipinyl-7-ACA.

**Revendications**

1. Utilisation de la transaminase de D-acide aminé, provenant de Bacillus licheniformis ATCC 9945, pour transformer la céphalosporine C, avec des α-cétoacides, en α-cétoadipinyl-7-ACA et en D-acide α-aminé correspondant.

2. Procédé de fabrication de la transaminase de D-acide aminé, caractérisé en ce que
   a) on fait fermenter, en milieu aérobie, Bacillus licheniformis ATCC 9945,
   b) on sépare la biomasse d'avec le milieu de fermentation et on la désagrège, et
   c) on isole la fraction qui transforme la céphalosporine en α-cétoadipinyl-7-ACA par réaction avec des α-cétoacides.

3. Utilisation de la transaminase de D-acide aminé, obtenue par fermentation de Bacillus licheniformis ATCC 9945, séparation et désagrégation des cellules, puis isolement de la fraction transaminase de D-acide aminé, pour transformer la céphalosporine C en α-cétoadipinyl-7-ACA par réaction avec des α-cétoacides.